Europäisches Patentamt

European Patent Office

Office européen des brevets

(11) Publication number: **0 127 650**
**B1**

## EUROPEAN PATENT SPECIFICATION

(45) Date of publication of patent specification: **15.03.89**

(51) Int. Cl.⁴: **G 01 N 23/223,** G 01 N 33/34

(21) Application number: **83903793.4**

(22) Date of filing: **30.11.83**

(86) International application number:
**PCT/FI83/00076**

(87) International publication number:
**WO 84/02191 07.06.84 Gazette 84/14**

(54) **PROCEDURE AND MEANS FOR MEASURING WITH THE AID OF AN X-RAY TUBE THE DISTRIBUTION OF FILLERS OR EQUIVALENT IN A WEB.**

(30) Priority: **01.12.82 FI 824142**

(43) Date of publication of application:
**12.12.84 Bulletin 84/50**

(45) Publication of the grant of the patent:
**15.03.89 Bulletin 89/11**

(84) Designated Contracting States:
**AT BE CH DE FR GB LI LU NL SE**

(56) References cited:
**DE-A-2 946 567**
**DE-A-3 219 962**
**DE-B-1 773 085**
**US-A-4 081 676**
**US-A-4 147 931**

(73) Proprietor: **Robotest Oy**
**Teknologiantie 1**
**SF-79480 Kangaslampi (FI)**

(72) Inventor: **KUUSI, Juhani**
**Pihlajatie 12-14 C 20**
**SF-00270 Helsinki 27 (FI)**

(74) Representative: **Berglund, Gustav Arthur et al**
**AWAPATENT AB Box 5117**
**S-200 71 Malmö (SE)**

(56) References cited:
**Paperi Puu 52, nr4a, p. 145-51, 154-8 (April 1970) J. Kuusi "Determination of content and distribution of filler and coating materials in paper using radioisotope X-ray spectrometry"**
**Abstract in English from Paperchem, accession Number 50-06512 Tsellyuloza Burmaga Karton nr. 3, 8 (1979) (Russ) Lienyanskii et al "Monitoring apparatus for determining filler content in a moving paper web"**
**Abstract in English from Paperchem, accession Number 46-03646 Sb. Tr. Ukp. NII Tsellyul - Bumazh, prom nr. 17: 1974 p 123-126 (Russ) Gladarevskii et al "Use of X-rays for determining the filler content in paper"**

**EP 0 127 650 B1**

## Description

The present invention concerns a method for measuring the distribution in the thickness direction of filler and/or coating materials of paper, cardboard or equivalent and the contents of said materials without destroying the sample, in said procedure the radiation emitted by an x-ray tube being used to excite in the material component under examination of the object under measurement its characteristic x-ray radiation, the intensity of this radiation being observed, and in which method measurements are carried out on both sides of the specimen under examination and in addition the contents of other filler components are determined by x-ray radiation absorption measurements, in order to eliminate the effects of said components interfering with the distribution measurement, and the base weight of the paper is determined by beta radiation absorption measurement or by another equivalent procedure.

When paper and paper machines are discussed in the following, reference is generally made both to paper and cardboard, and respectively both to paper and cardboard machines.

Fillers, which as a rule are mineral substances, are incorporated in the paper primarily for their effect of improving the printing technological properties. Fillers are most commonly used for printing papers. The filler addition improves the opacity, lightness, printer's ink absorption and surface smoothness of the paper.

The fillers influence in a particularly advantageous manner the quality of paper to be glazed.

It is known in the art to add filler material in two ways, either by mass filling or by coating. In the former procedure, the filler material is added in the form of suspension to the pulp sludge before the arrival of the sludge on the paper machine, whereby the filler material is admixed to the entire fibre material in the finished paper. In the latter procedure, a suitable glue substance is admixed to the filler material in the aqueous phase, such as starch or casein, wherefter the surface of the paper is brushed with this mixture in a continuous process.

The filler materials in paper tend to be non-uniformly distributed in the thickness direction of the paper, causing one-sidedness of the paper. The one-sidedness of paper manufactured on Fourdrinier machines is due to the fact that the fillers are washed out together with the water that is drained, from the lower part of the pulp web into the drainage water, whereby they become enriched in the upper part of the web. As is known in the art, endeavours have been made to reduce the problems of one-sidedness, not only by additives improving the retention, but also by gentle dewatering at the initial draining phase, which requires a longer dewatering time and therefore implies lengthening the wire section or reducing the speed of the paper machine.

In machines with a planar wire, the difficulties with the fines and filler distribution manifest themselves when papers for offset printing are manufactured. A high filler and fines content on the top surface of the paper causes dusting, which is a serious detriment in the offset process. In contrast, papers manufactured on a twin wire machine are considered well appropriate for offset printing. This is due to the symmetrical shape of the fines distribution and to equal leaching of both surfaces of the web owing to two-sided dewatering. It is in fact generally held that owing to more uniform fines distribution, the printing by offset on paper manufactured on a twin wire machine is more successful than that on paper manufactured on a Fourdrinier machine. Offset printability has indeed increased in significance because offset printing is increasingly replacing the letterpress printing procedure.

On the other hand, the filler content of the surfaces of the paper web cannot always be brought on desired level with a twin wire former, and even when using planar wires only the top side of the web (the side facing away from the wire) is satisfactory as to its filler content. The low filler content of the web surfaces is particularly problematical in so-called SC gravure papers. Attempts may be made to increase the filler content of the paper surfaces by increasing the filler content of the pulp in the headbox, but even with this expedient no satisfactory condition is achieved because of the above-mentioned poor retention characteristic of the filler and of its enriching in the inner parts of the paper. In addition, when the filler content in the headbox has to be increased, the consistency in the headbox is likely to become excessive so that it impairs the formation of the paper.

Modern high-speed printing presses impose particularly high requirements on the printing paper. These requirements are based on trouble-free operation of fast printing presses and on the appearance of the printing. The imprint is considerably influenced by the symmetry between the sides of the paper and the quality of the surfaces of the paper, which is naturally also influenced by the distribution of the fillers. Heretofore no procedures and means have been in use with which the filler distribution could have been measured even on line either on the paper machine, on the printing press or on the paper coating means. The object of the present invention is therefore to provide a new procedure and means suited, in addition to laboratory measurements, for the uses mentioned and which renders possible the control and adjustment of the manufacturing process on the paper machine on the basis of filler distribution measurements.

It is known in the art (e.g. the Finnish Patent No. 40587; inventors: Juhani Kuusi and Antti Lehtinen, applicant: Valmet Oy) to excite the characteristic x-ray radiation of the filler material by means of radiations (alfa, beta, gamma or x-ray radiation) penetrating into various depths in the paper, and in this way to gain information on the vertical distribution of the filler. The procedure has been described in greater detail in: J. Kuusi, Determination of Content and Distribution of Filler and Coating Materials in Paper Using Radioisotope X-Ray Spectrometry, Paper and Timber No. 4A (1970) p. 145—158. All the examinations in the

2

paper were based both on experimental x-ray fluorescence intensities measured from both sides of the paper sheet and on calculated intensity ratios, which were estimated from known paper filler or coating material distributions. The examinations concerned circumstances in which paper filler or coating material was composed of only one single filler or a mixture of constant composition. Variations in relation to each other of the filler component contents as is frequently the case in practice cause effects on the measured x-ray fluoroscence intensities, the quantitative elimination of which is impossible by the procedures described in the paper. This has impeded the introduction in practice of the procedures.

As regards filler measurements, the state of art is illustrated in general by the publication of April 1982; Buchnea A., McNelles L. A., Hewitt J. S., The Application of X-Ray Absorption and Fluorescence Analysis to the Measurement of Paper Additives, Int. J. Appl. Radiat. Isot. Vol. 33, pp. 285 to 292 (1982), where a fluorescence and absorption technique is used to the purpose of determining the total contents of different fillers. The filler component total contents are determined by x-ray fluorescence measurements except for the one which has too low characteristic x-ray energy and which is measured by the x-ray absorption measurement. The measurements are based on the assumption that the fillers are uniformly distributed in the thickness direction of the paper. In practice, this is hardly ever the case. It is thus understood that in said publication no endeavours whatsoever have been made to determine the important thickness-direction distribution, nor has it ever been taken into account as a potential source of error in determination of the total filler content. It should be noted, however, that in the instances described in the paper the influence of said source of error is minimal.

The object of the present invention is to provide a procedure and apparatus by which the thickness-direction filler distribution in the paper and the total filler contents can be determined either in the laboratory or directly on the paper machine (on line) also in the case that the contents of different filler components, e.g. $CaCo_3$, $TiO_2$, kaolin, talc or equivalent, are variable.

Procedures capable of determining the filler distribution and the total filler content directly on the paper machine are not in use at all. The object of the present invention is to provide an opportunity not only for immediate product quality control directly on the machine (on line), but also an entirely new possibility of controlling the paper manufacturing process, the significance of which is emphasized when endeavours are made to manufacture printing paper meeting even greater quality requirements at lowest material cost. The object of the present invention is to achieve that the distribution can be measured and that it can also be controlled, which opens also a chance to develop the paper machine construction and the total control systems of paper machines.

In addition, it is an object of the invention to provide a procedure which is also suitable for quality control of the paper fed into fast modern printing presses, and possibly for the control and/or adjustment of the operation of said printing presses.

The method defined in the foregoing may be used, as claimed in claims 13—16, e.g. on a paper machine, in on-line measurement for measuring the filler distribution in the thickness direction and the total filler content of paper. In addition, the results of measurement that are obtained can be used as feedback signals in the control system of the paper machine in the control of the filler distribution and/or of the total filler content of various filler materials. An advantageous application of the invention is in measurement, and possibly in the control, of the coating material content and/or coating material distribution in paper or cardboard that is either being coated in an on-line process or has been tested in a separate coating apparatus, in particular of its one-sidedness.

One potential application of the invention is the quality control of the paper being fed into a printing press and/or controlling, the operation of the printing press.

As has in part become apparent already in the foregoing, the inventive idea is that on both sides of the paper is measured the intensity of the characteristic x-ray radiation of the filler component excited with different radiation sources and possibly with different angles of incidence of the exciting radiation, this intensity furnishing information about the shape of the distribution. In addition to this it is possible in this x-ray fluorescence measurement to determine, to serve as an auxiliary quantity in the processing of results, the intensity of the exciting radiation scattered back from the paper and which correlates, for instance, with the base weight of the paper. What is significant from the point of view of practical applications is that the contents of various filler components are measured by means of x-ray absorption mesurements, making use of the primary radiation, possibly varying as to its energy with reference to time during the measuring cycle, emitted by the x-ray tube and a radiation with desired absorption properties which has been derived therefrom or from an x-ray tube placed on the other side of the paper, with the aid of appropriate transformation targets. The auxiliary quantity is the absorption measurement signal of beta radiation used as routine in measurements on paper for determinations of base weight (in $g/m^2$) (fibres plus filler). Based on the results of the absorption measurements, it is possile by calculation to eliminate the effects of the variation of the different filler components' contents on the fluorescence measurements, and in this manner to determine the filler distribution and the contents of different filler components.

In the laboratory, the invention affords a chance for a rapid quality control of the paper, and thereby for the control of the manufacturing parameters with a given lead time. Particularly the filler distribution close to the surface layers of the paper has a remarkable significance for the printability of the paper. Moreover, a distribution of proper shape gives an opportunity to use filler in abundance, thereby lowering the total material costs. The procedures presently used in laboratories, such as dividing the paper into different

# EP 0 127 650 B1

layers by the aid of a tearing tape, incineration of layers and ash determination, are slower by one order of magnitude and more inaccurate than the procedure of the present invention.

Various embodiment examples of the invention and its physical and mathematical background are considered in the following in greater detail, reference being made to the figures of the drawing attached, to the details of which the invention is not confined.

Fig. 1 presents a typical filler distribution in paper manufactured on a Fourdrinier machine.

Fig. 2 presents mass absorption coefficients of some mineral filler and coating materials of paper and of water and of cellulose for low energy x-ray radiation.

Fig. 3 shows the main principle of the fluorescence measurement of the invention.

Figs. 4A and 4B present the principle of the fluorescence measurement of the invention with two different angles of incidence of the exciting radiation and angles of departure of the excited radiation.

Fig. 5A presents the variation of the average energy of the radiation emitted by an x-ray tube used in accordance with the first embodiment of the invention during the measuring cycle, as a function of time.

Fig. 5B shows the count frequency recorded by the counter connected to the detector, in absorption measurements during one measurement cycle, as a function of time.

Fig. 5C shows the count frequency of the fluorescence signals (Ca K line) during one measuring cycle as a function of time. The graph in Fig. 5C drawn as a solid line represents the result of measurement obtained from the top side of the paper shown in Fig. 1, and the interrupted line represents the corresponding result of measurement obtained on the wire side.

Fig. 6A presents the distribution of filler components prior to coating the paper, while Fig. 6B presents the same paper after the coating process.

Fig. 7A illustrates a means of the invention and its measuring head wherein an x-ray tube emitting constant energy radiation is applied.

Fig. 7B shows an arrangement by which absorption measurements are carried out with x-ray radiation of different energies.

Fig. 7C presents a measuring apparatus and its measuring head wherein an x-ray tube emitting radiation of varying energy during the measuring cycle is applied.

Fig. 7D illustrates the beta absorption measurement by which the auxiliary quantity required in the invention is produced.

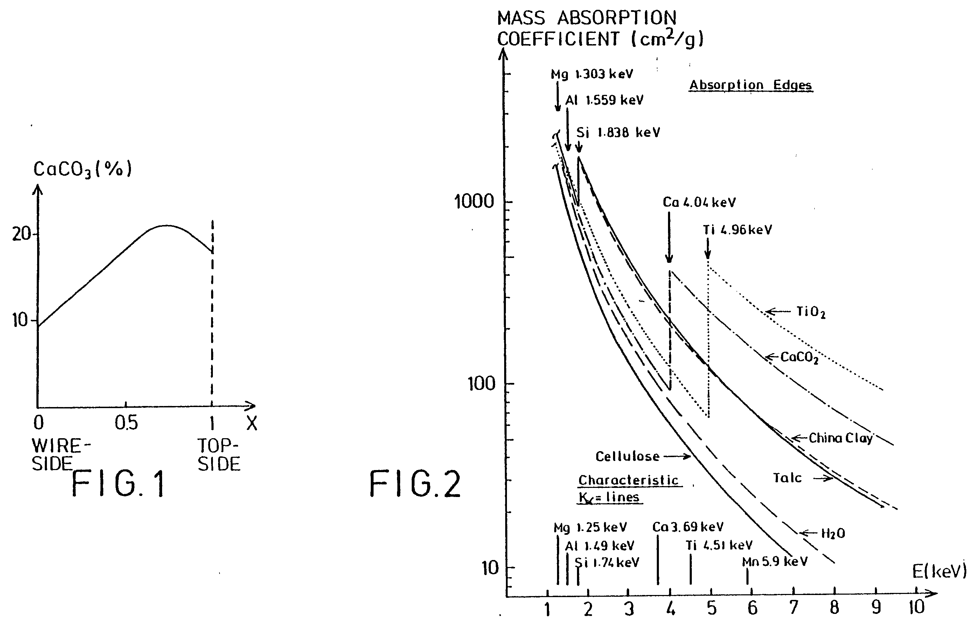

A typical filler distribution of paper in its thickness direction x can be seen in Fig. 1. The filler is least in quantity on the wire side. In this instance, the maximum is reached slightly above the centre-point of the paper (marked as 0.5 on the horizontal axis). The filler content decreases towards the top surface (x = 1).

The attenuation (extinction) of x-ray, gamma and beta radiation in matter can generally be expressed by the exponential formula:

$$1 = i_0 \, e^{-\mu m}$$

where I (I/s) is the intensity of the radiation that has gone through a mass course m (g/cm$^2$), $I_0$ (c/s) the original intensity of the radiation and $\mu$ (cm$^2$/g) is the absorption coefficient representing the extinction capacity of the material.

The absorption coefficients for low energy (1 to 10 keV) x-ray and gamma radiation (both are the same kind of electromagnetic radiation) of materials which are important in view of filler measurements are set forth in Fig. 2, plotted over energy. In the figure, the energy (in keV) has been plotted on the horizontal axis and the absorption coefficient $\mu$ (in cm$^2$/g) on the vertical axis. With the exception of a few discontinuous irregularities, the absorption coefficient and therefore also the extinction in the material decreases with increasing energy. However, some of the discontinuous jumps seen in the figure are not of central importance in the embodiments of the invention. If we scrutinize the graph of the absorption coefficient of calcium carbonate (CaCO$_3$), we find that it descends smoothly throughout the range from 1 to 4 keV, until at 4.04 keV energy its value discontinuously increases to be tenfold and thereafter once more decreases smoothly with increasing energy of the radiation. The physical cause underlying this jump is as follows: in the range under consideration, x-ray and gamma radiation are attenuated in the material in the way that the energy of the radiation quanta transfers totally to electrons in the atoms, such electrons by virtue of the energy imparted to them being flung out from the atom, leaving behind a vacancy in the electron shroud. The energy of the x-rays or gamma quantum has to be higher than the binding energy holding the respective electron to its atom. When the energy of the radiation is lower than the 4.04 keV corresponding to the jump in the graph for CaCO$_3$, the radiation is not able to detach from the calcium atom the electrons of its inner shell (the K shell), which are the electrons most strongly bound to the atom. When the energy of the incident radiation surpasses this limit, its quanta can become absorbed in the substance by detaching electrons from the inner shell, and this exactly gives rise to the discontinuous increment of the absorption coefficient. The higher the atomic number of a substance — in practice usually the heavier it is — the higher is the energy at which is found this K absorption limit, i.e. the absorption limit corresponding to the K shell.

Thus, it is shown in Fig. 2 that the K absorption limit, due to titanium, of titanium dioxide (TiO$_2$) is located at the energy of 4.96 keV. In talc and kaolin, the element with the highest atomic number is silicon (Si), and therefore the absorption coefficient decreases steadily after the absorption limit of silicon at 1.8 keV with increasing energy of the radiation.

4

It is thus understood that when on a substance, for instance on calcium, radiation is directed which has an energy higher than the K absorption limit of calcium, vacancies will form on the inner electron shells of the atoms. When these are filled by electrons falling from outer shells, the substance emits its characteristic K x-ray radiation, the energy of which because of recoil losses is slightly lower than the energy of the K absorption limit. The strongest line of the calcium K has energy 3.69 keV, which has also been indicated on the energy axis in Fig. 2.

Said characteristic x-ray radiation of each element produced through absorption is utilized in a way known in the art in x-ray fluorescence analyses for determining the chemical composition of the specimens being analyzed. In the present invention, said absorption is utilized towards determining the filler content of the paper's different layers and thus towards determining the filler distribution. In order that the determination of said distribution could be made free enough of error from the viewpoint of the practical applications, the total contents of the different filler components in the paper must be known. This is found out, in the present invention, by the aid of absorption measurements.

If in the absorption measurements the extinction caused by paper containing filler is measured with two radiation energies which are as close as possible to the absorption limit of a given component in the way that one energy is above and the other below the limit, the difference in the extinction caused by the paper will furnish information about the content of this filler component. If the paper contains kaolin, talc, calcium carbonate and titanium oxide as fillers, the difference in the extinction of the K line of manganese (5.9 keV) and of the K line of titanium (4.51 keV) will furnish information primarily about the titanium dioxide content (Fig. 2), the difference in the extinction of the differences of the 4.51 keV (Ti K) and 3.69 keV (Ca K) radiations will furnish information primarily about the $CaCO_3$ content, and the absolute extinction of the 3.69 keV radiation, primarily about the combined content of talk and kaolin, these latter components having absorption components which at the last-mentioned point are clearly higher than the absorption coefficients of any other components of the paper, as Fig. 2 reveals.

For determining the contents of various filler components of paper, it is furthermore necessary to know the base weight of the whole paper, that is, its mass per unit area (in $g/m^2$). This is found by measuring the extinction in the paper of beta radiation e.g. from an $^{85}Kr$ source. This is because the different components of paper cause equal extinction of beta radiation (i.e., of electrons thrown out by nuclei). The use of beta radiation for determining the base weight of the paper is known in the art of paper technology and is altogether routine of its nature.

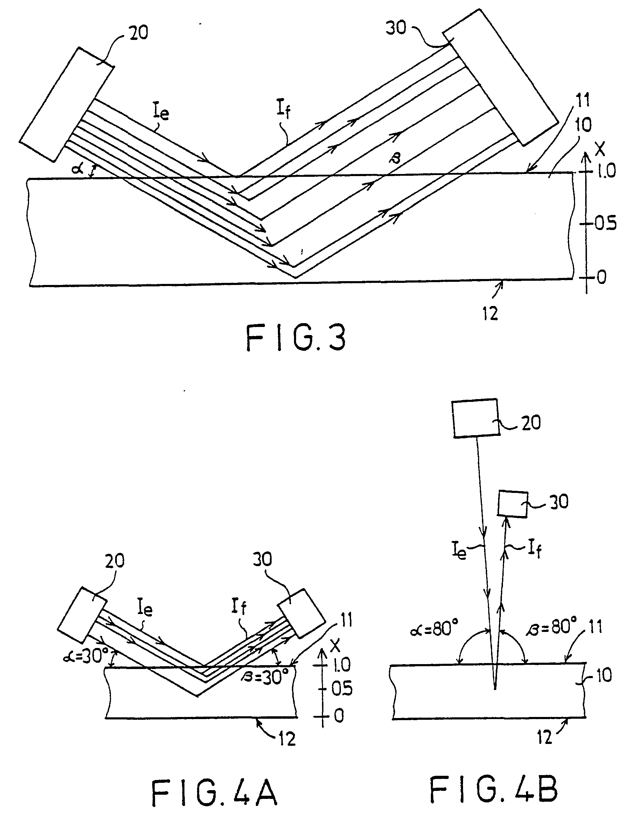

The fluorescence measurement used for the actual determination of the filler distribution is described more closely in the following referring to Fig. 3, in connection of which it shall be assumed that the base weight of the paper specimen 10 is 100 $g/m^2$ and that it contains, as uniformly distributed filler, 25% calcium carbonate. As shown in Fig. 3, the exciting radiation $I_e$ from the source 20, which is an x-ray tube, impinges on the paper specimen 10 under the angle of incidence a and excites in the specimen 10 the characteristic radiation of calcium, of 3.69 keV. The detector 30 measuring this radiation $I_f$ observes the radiation departing under the angle β from the surface 11 of the specimen 10. Since the exciting radiation $I_e$ is attenuated as it proceeds in the paper specimen 10, it excites calcium radiation more efficiently in the adjacency of the top surface 11 which lies closer to the source 20 than in the adjacency of the lower, or back, surface 12. Since the excited characteristic radiation of calcium also suffers extinction in the specimen 10 to a given extent, the radiation excited adjacent to the top surface 11 has easier access to the detector 30. Both circumstances just mentioned act in the direction that the greater part of the radiation detected by the detector 30, in the case of the homogeneous filler distribution, comes from the top layers of the specimen 10, and therefore the topmost layers of the paper will be emphasized in the information thus obtained. The smaller the angles of incidence and departure a and β of the radiation, the greater are the differences in path length between the top surface and the lower surface 11 and 12, and the greater is the stress placed on the top surface 11 in the information gained by the detector 30. In this manner, it is possible by varying the angles of incidence and departure a and β to change the relative weight factors of different layers in the information that is measured. This is demonstrated by Figs. 4A and 4B and by the following Table 1.

TABLE 1

| | | 80° | 30° |
|---|---|---|---|
| Angle of incidence of the radiation (a) | | 80° | 30° |
| Angle of departure of the radiation (β) | | 80° | 30° |
| | Depth | Intensity | Intensity |
| Relative intensity of information from different depths in the paper | 0.05 | 0.93 | 0.86 |
| | 0.5 | 0.47 | 0.22 |
| | 0.95 | 0.23 | 0.06 |

In the above Table 1 is shown the relative intensity of the information received in fluorescence measurements at various depths in the specimen when two different pairs of angles of incidence and of departure α, β of the radiation are used. The energy of the exciting radiation is equivalent on the average to the K line of manganese (5.9 keV). The base weight of the paper is 100 g/m$^2$ and its CaCO$_3$ content 25%, assumed in this calculation example to be uniformly distributed in the vertical direction. On the depth scale, the surface has been denoted with the coordinate 0 and the back side of the paper with the value 1, making the coordinate of the centre 0.5.

The intensity values calculated in Table 1 reveal that the information is strongly weighted in favour of the top side, in other words, emphasizing that side, on which the measurement is performed. On changing the angles of incidence and departure of 80° in Fig. 4B one transfers to the angles α, β of 30° in Fig. 4A, this effect is even considerably strengthened. This is seen when, for instance, the values of the intensities obtained from the centre of the paper (0.5) are mutually compared (0.47 and 0.22).

Another way in which the relative weighting of different layers of the specimen 10 can be varied is to change the energy of the radiation used for excitation, i.e., of the radiation emitted by the x-ray tube 20. When this is done in regular manner within a given measuring cycle, the fluorescence and absorption information required for determining the distribution is obtained simultaneously. This case will be examined more in detail later on.

If now the distribution of a given filler component in the thickness direction of the specimen 10 is not uniform but for instance like that shown in Fig. 1, the intensities of the characteristic radiation of calcium measured on different sides of the paper are unequal and their difference reflects the one-sidedness of the distribution. In the case of a paper having a distribution substantially like that of Fig. 1 and with base weight 160 g/m$^2$ and calcium carbonate content about 20%, with 5.9 keV radiation and using angles of incidence and of departure α,β = 80° on the average, for the ratio of the intensities on different sides (top side/wire side) was found: 470/410. When the angles of incidence and of departure were reduced, the ratio increased as could be expected. An effect in the same direction was achieved by using softer radiation (4.5 keV).

In the following, the determination of the filler distribution on the basis of the results of measurement shall be considered.

The basic distribution as in Fig. 1 can be mathematically represented by a polynominal $y = ax^2 + bx + c$, where y refers to filler content (vertical axis) and x to the coordinate in the vertical direction of the paper (horizontal axis). The coefficients a, b and c are found by fitting to a reference distribution. From a paper with reference distribution, the intensities of the characteristic radiation of calcium are determined from both sides of the paper to serve as reference values, and the x-ray absorption of the paper at a suitable energy, and the beta absorption (e.g. $^{85}$Kr source).

When now from an unknown specimen belonging to the same paper brand are measured the equivalent quantities, the differences between them and of the quantities measured from the reference paper will yield the filler distribution of the paper sample that is being measured, by mathematical methods, utilizing the known absorption coefficients of the different components. In the vicinity of the reference distribution, a measurement carried out with merely one pair of angles α,β or with one energy of the exciting radiation already yields a rather reliable estimate on the distribution. The reliability and accuracy can be increased by varying the angles of incidence and of departure α,β or by using several different radiation energies. This naturally causes the mathematical processing to be more complicated.

In a case which was studied, for the reference polynominal representing the filler distribution was found $y = -42x^2 + 51.1x + 6.7$, the unit of y and of the coefficients a, b and c being the CaCO$_3$ content (in %). It follows that the CaCO$_3$ according to the reference distribution, on the wire surface 12 (x = 0) of the specimen 10, is 6.7% and on the top surface 11 (x = 1), respectively, is 16.8%.

After the results of measurement for the intensity (I) of the characteristic radiation of the calcium ($I_1$ is the wire side 12, $I_2$ is the top side 11) and the result of the x-ray absorption measurement (T) for the paper specimen under examination have been corrected by applying the reference graph, by the aid of the results of the beta absorption measurements to correspond to the base weight of the reference paper, the changes ($\Delta a, \Delta b, \Delta c$) of the coefficients of the distribution polynominal for the paper under examination can be calculated from the system of equations calculated from the reference polynominal:

$$\frac{\Delta I_1}{I_1} = 0{,}6113 \cdot \Delta a + 1{,}127 \cdot \Delta b + 3{,}344 \cdot \Delta c$$

$$\frac{\Delta I_2}{I_2} = 1{,}0403 \cdot \Delta a + 1{,}832 \cdot \Delta b + 2{,}781 \cdot \Delta c$$

$$\frac{\Delta T}{T} = \frac{1}{2} \cdot \Delta a + \frac{1}{2} \cdot \Delta b + 1 \cdot \Delta c$$

6

In the system of equations, $\Delta I, \Delta Ia$ and $\Delta T$ correspond to the values of the paper specimen 10 under examination and to those measured from the reference paper.

In tests that have been carried out, the new coefficients obtained from the system of equations were found to yield distributions in agreement with the distributions determined from the same paper specimens by activation analysis close to the reference distribution. It is obvious that a more accurate approximation is attained by a greater number of measurements, but the accuracy afforded by the procedure described in the foregoing is adequate in certain supervision applications.

If in the instance we are now considering, to the filler of the paper is added for instance kaolin in addition to calcium carbonate, as is frequently done intentionally or inadvertently (reused paper, etc.), the situation is significantly altered in the sense of measuring technology. This is because kaolin attenuates, in fluorescence measurements, both the exciting $I_e$ and the excited $I_f$ radiation (especially the last-mentioned), and as result the variations of kaolin content affect to a certain degree the calcium carbonate measurements, even if the content and distribution of the latter should be constant in the specimen 10. The influence of kaolin on the results is however calculable and can be eliminated by the aid of the known absorption coefficients, provided that the kaolin content in the specimen 10 is known. This leads to the requirement of measuring technology that in connection with the measurements the contents of kaolin and other potential filler components have to be determined. This is possible by using in the absorption measurements radiation energies suitably selected, as has been described in the foregoing. It may be observed in this connection that of the commonly used fillers, talc and kaolin are materials of which the contents must be determined by the absorption technique. Fluorescence measurements do not succeed in normal conditions because in these substances the characteristic x-ray radiation even of the heaviest element, silicon (Si), is so weak that it is excessively attenuated in the specimen 10, in the air space and in the windows of standard detectors 30. Regarding $TiO_2$, which is occasionally used, the same methods can be applied as for $CaCO_3$, with the difference of course that the K line (4.51 keV) of titanium is excited and measured.

It is thus understood that in complicated cases the determination of the thickness-direction distribution of filler requires several x-ray fluorescence measurements on both sides of the specimen 10 and several absorption measurements. The intensity of the exciting radiation $I_e$ scattered back from the specimen 10, which correlates with several characteristics of the specimen paper may be used as a kind of control quantity in the measurements. In practical instances, when one is moving quite close to a given reference distribution, adequate accuracy is often achieved with rather few measurements.

If the x-ray tube 20 is so constructed that the energy of the radiation therefrom can be varied during a measuring cycle (T) and at the same time the change of the measuring signal (pulses per unit time) can be recorded as a function of the energy of the exciting radiation, the measuring event can be considerably simplified.

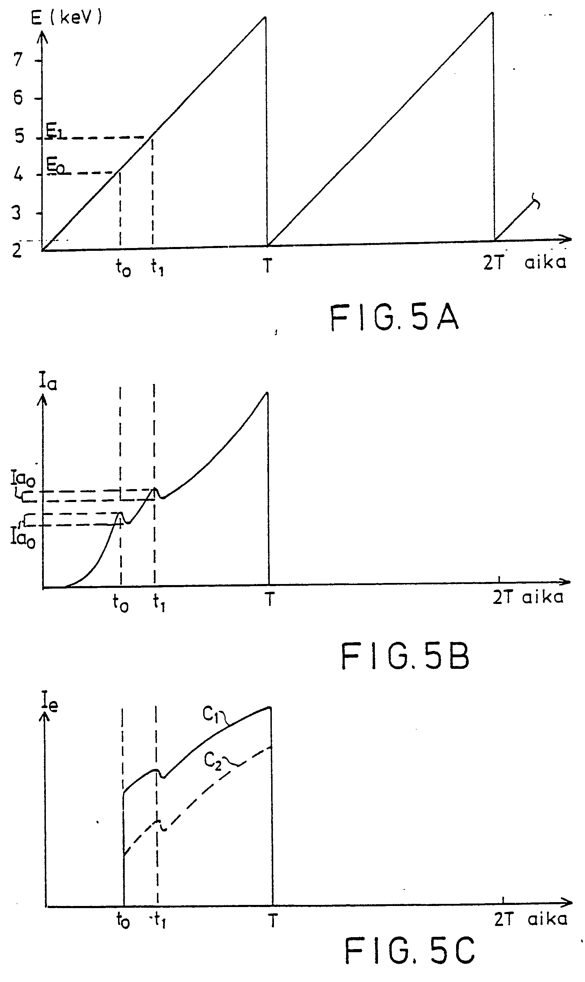

The energy of the radiation emitted by the x-ray tube 20 changes during one cycle of measurement advantageously, e.g. as shown in Fig. 5A. If the paper specimen 10 contains as fillers kaolin, talc, calcium carbonate and titanium oxide, the pulse frequency observed by the counter 42 will vary in the radiation absorption measurement as shown in Fig. 5B. When at the beginning of the cycle T the energy reaches a certain technical limit threshold, the counter 42 starts to observe pulses, and the count frequency increases uniformly at first, with increasing energy. When the energy reaches the K absorption limit of calcium $E_0 = 4.04$ keV, the count frequency falls after the respective time $t_0$, owing to the discontinuously increasing absorption coefficient of calcium. Thereafer, with further increase of the energy of the radiation, the count frequency falls smoothly until the K absorption limit $E_1 = 4.9$ keV of titanium is reached, after the respective time $t_1$ a drop of the count frequency ensuing, caused by the discontinuous increase of the absorption coefficient of titanium. By using the magnitude of the step $E_1 - E_0$ (Fig. 5A) and the differences of count frequency $Ia_0$ and $Ia_1$ (Fig. 5B), and the absorption measurement carried out by the aid of beta radiation (e.g. $^{85}Kr$), the base weight of the paper specimen 10 is found and the contents of calcium carbonate and titanium dioxide and the combined contents of talc and kaolin, for the corrections by calculation of the fluorescence measurements required in the determinations of distribution. In practice, it is difficult or even impossible to alter the energy of the radiation emitted by the x-ray tube 20 in such an ideal and monochromatic way as is shown in Fig. 5A. If however the pulse frequencies $Ia$ recorded by the counter 42 on the average correspond to energies between which the absorption jumps fall, the results will, with appropriate calibration measurements, usually furnish the filler contents with sufficient accuracy.

In the fluorescence measurements constituting the basis for the determinations of distribution, the count frequency $I_e$ of the counter 42 changes during the measuring cycle as is shown in Fig. 5C. The filler distribution is assumed to be still as in Fig. 1, and the object of examination is the observed intensity of the calcium K line excited in the paper specimen. The solid-line graph $C_1$ in Fig. 5C corresponds to the measurement from the top side of the paper specimen (x = 1), and the graph $C_2$ drawn with an interrupted line $C_2$ similarly represents that from the underside of the paper. When at the beginning of the meaasuring cycle T the energy of the exciting radiation is lower than the K absorption limit of calcium, no characteristic radiation of calcium at all will of course be produced in the paper. When this limit is just surpassed at the time $t_0$, the signal measured on the top side of the specimen (x = 1) will in accordance with Fig. 5C rise to be clearly higher than the signal measured on the wire side (x = 0), because soft radiation "sees" more calcium on the top side. As the energy of the radiation increases, the signals measured on the top side and

7

on the wire side (graphs $C_1$ and $C_2$) approach each other, however so that even with high exciting radiation energies the signal measured on the top side remains higher than that obtained from the wire side, owing to the absorption of excited radiation taking place in the paper. At the point ($t_1$) corresponding to the K absorption limit of titanium $E_1 = 4.96$ keV there is a small dip in the graphs $C_1$ and $C_2$ by reason of the increasing matric absorption.

The pulse frequencies $I_e$ selected at a suitable point in Fig. 5C correspond to the count frequencies $I_1$ and $I_2$ excited with constant energy on different sides of the paper in the preceding examples, and they can therefore be used in the mathematical procedures presented for determining the distributions. This measuring technique has the advantage that during the measuring cycle a great number of such intensity pairs ($I_1$, $I_2$) is obtained as a function of the exciting radiation, by the aid of which more detailed information about the course of the distribution is obtained by more advanced mathematical considerations. Furthermore, it is naturally necessary to carry out corrections for eliminating the effects from the filler components' variations in relation to each other on the basis of the total contents determined by absorption measurements. As was mentioned in connection with the absorption measurements, it is difficult or even impossible in practice to change the energy of the radiation emitted by the x-ray tube 20 in such ideal and monochromatic manner during one measuring cycle as has been shown in Fig. 5A. However, even with a less ideal cycle T, in which intensity pairs are obtained corresponding to a few average energies, adequate information is in practice obtained for determining the distribution.

As the preceding consideration reveals, an exciting energy E varying within the measuring cycle T supplies considerably more information about the distribution and about the contents of the filler components than an exciting source with constant energy. However, the first mentioned imposes rather more exacting demands on the electronics of the measuring apparatus, in that it is necessary in this instance to record a continuously varying count frequency during the cycle T, and one cannot be content with simply counting the accumulated number of pulses.

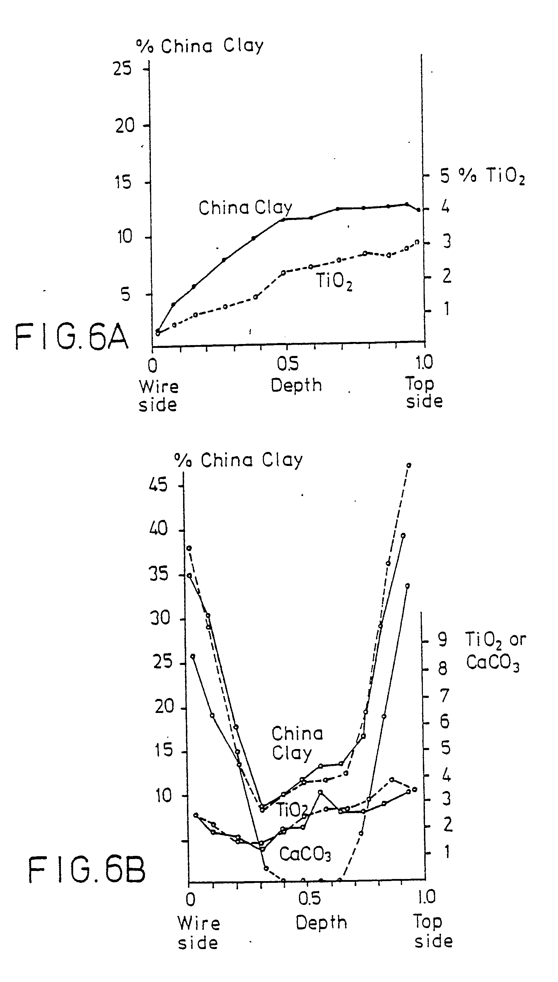

The printing characteristics of paper can be improved by coating the paper with the same substances that are used also as fillers. In that case the contents of mineral components in the surface layers of the paper increase greatly, as can be seen in Figs. 6A and 6B already discussed. Since by the procedure of the invention information is gained about the distribution of the papers' mineral components in the paper and in particular about their content in the surface layers of the papers, it is also possible to determine the amount of coating in the coating layers and the difference in coating between the different sides of the paper by the procedure of the invention without destroying the specimen. If the paper is already coated, the filler distribution of the uncoated bottom paper naturally cannot be elicited any longer.

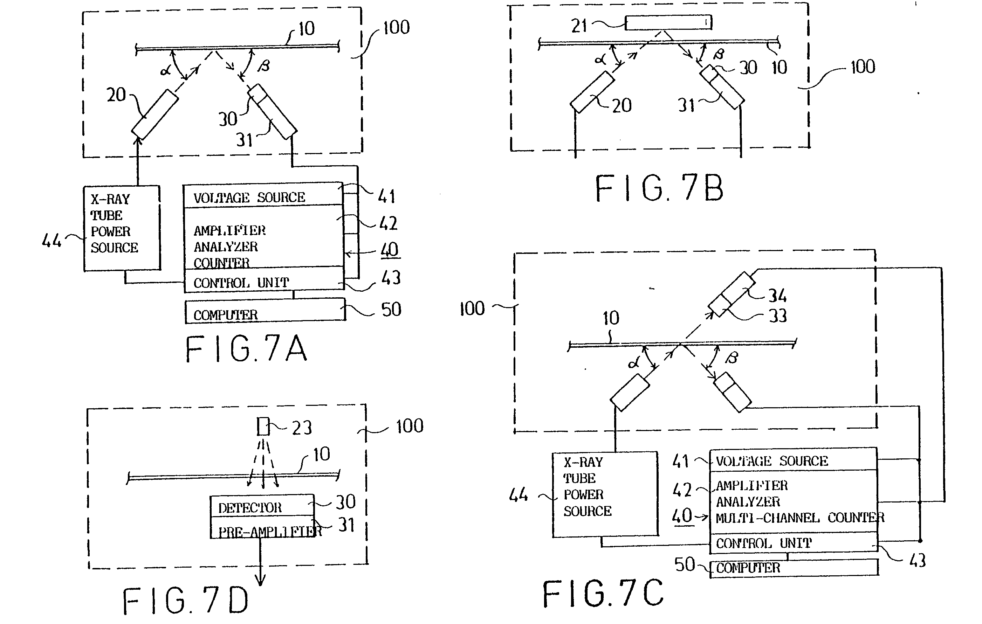

The measurement arrangement and apparatus of the invention are presented in Figs. 7A, 7B and 7C. Fig. 7A illustrates the x-ray fluorescence measurement by an x-ray tube 20 emitting radiation of constant energy. Fig. 7B shows the equivalent absorption measurement. It is naturally required that a fluorescence measurement is performed on both sides of the paper. In Fig. 7C is shown the measurement carried out with an x-ray tube 20 emitting radiation varying in energy within the measuring cycle T as shown in Fig. 5A. The fluorescence measurement on one side of the paper and the absorption measurement can now be accomplished simultaneously. In this instance as well, a separate fluorescence measurement has naturally to be carried out on the other side of the paper.

The part 100 isolated with interrupted lines in Figs. 7A, 7B and 7C is the measuring head, comprising the x-ray tube 20 with a mechanism, if any, for changing the angle of incidence of the exciting radiation $I_e$, a detector 30 with pre-amplifier 31, and in the case of an x-ray tube 20 emitting constant energy radiation also a transfer mechanism for the radiation transformation target. In a laboratory apparatus, the measuring head 100 is for instance an enclosed apparatus on the table, into which the paper specimen 10 to be examined is conveyed by a suitable mechanism. In an on-line apparatus accomplishing the measurements directly on the paper machine, the paper web passes through the measuring head 100 mounted on a measuring beam. The measuring head 100 may be so constructed that it can be traversed across the paper web.

For detector 30 is principally used a proportional counter or a scintillation crystal. In certain instances, in particular in laboratory measurements, a semiconductor counter may also be used with a view to increasing the accuracy.

When an x-ray tube emitting constant energy radiation is used (Figs. 7A and 7B), the measuring head 100 is connected to a measuring apparatus comprising a power source 41, an amplifier 42 and a counter, processor and display unit 42, and to the power and control unit 44 of the x-ray tube. A control unit 43 connected to a processor or computer 50 controls the performing of measurement and the processing of results. In the laboratory version, the processor operations may of course be replaced with manual operations and also the results may of course be processed manually or using an external computer. When using an x-ray tube 20 emitting radiation varying in its energy during the measuring cycle T, it is necessary to use instead of the counter 42 a multi-channel counter applying a time axis.

The extent of the equipment external to the measuring head 100 and of the programmes for the computer 50 that may be connected therewith is greatly dependent on the degree of automation and the standard of accuracy aimed at, and on the extent of the measuring range (on the number of different paper brands and the variation limits, within each brand, of the quantities which are measured).

Fig. 7A illustrates the exciting of the characteristic fluorescence radiation of a filler components ($CaCO_3$

or $TiO_2$) and its measuring on one side of the paper specimen 10. The radiation $I_e$ emitted by the radiation source 20 excites in the paper specimen 10 the characteristic x-ray radiation of a given element (Ca or Ti) of a filler, part of which is going to the detector 30 and being counted. The detector 30 differentiates between the different kinds of radiation by their energy with such accuracy that from the measured pulse height distribution the contribution of each radiation component can be elicited by mathematical means. If it is desired to make the measurement with different angles of incidence and departure, of the radiation with reference to the surface of the paper specimen 10, movable collineators or radiation beam detectors may be used.

Since for determining the distribution a fluorescence measurement has to be made on both sides 11 and 12 of the paper, in the laboratory version the paper specimen 10 must be turned over, or two measuring heads 100 carrying out measurement on different sides of the paper have to be used. When measurements are carried out directly on the paper machine, the latter alternative is the only possible.

Fig. 7B presents an arrangement by which absorption measurements are carried out with x-ray radiation of different energies. The radiation from the x-ray tube 20 that has passed through the paper specimen 10 excites in the backing plate 21 a radiation suitable for absorption measurements and which passes partly through the paper specimen 10 to the detector 30. In this instance to the signal being measured is admixed the signal of the radiation excited in the paper specimen 10 by the source, this signal reducing the accuracy of measurement in certain cases.

Fig. 7C represents the measurement performed with an x-ray tube 20 emitting during the measuring cycle radiation of varying energy, in which measurement the measuring of fluorescence on the same side of the specimen 10 where the x-ray tube 20 is located and the absorption measurement on the opposite side of the specimen 10 using the absorption detector 33 and a pre-amplifier 34 connected thereto can be accomplished simultaneously.

Fig. 7D presents the beta absorption measurement used as routine in the papermaking industry for base weight measurements and which in the distribution measurements supplies the auxiliary quantity indispensable in the processing of the results. These auxiliary measurements are accomplished using a beta source 23, a detector 30 and a pre-amplifier 31 in the manner known in itself in the art.

Detailed reference distributions which are indispensable for demonstrating and proving the practical applicability of the procedure of the present invention may be determined by neutron activation analysis of microtome sections made of paper. The technique is described in: Kuusi J. and Lehtinen, A. J., "Neutron Activation Analysis of Microtome Cuts in Examination of Paper for Its Filler Distribution", Pulp and Paper Magazine of Canada, 71, No. 3 (1970).

The procedure and the means described in the foregoing are suitable to be used either in laboratory measurements or in on-line measurements on a paper machine. In the last-mentioned use, the results obtained with the measuring apparatus may be used as feedback signals for guiding and/or controlling the papermaking process towards implementing a given desired filler distribution. A possible application of the invention is the use of the procedure or of the means in the measurement, and possibly even in the control, of the coating agent content and/or coating distribution either of paper or cardboard to be coated in an on-line process or of paper treated in separate coating means, in particular of its one-sidedness. Of the applications of the invention may further be mentioned the quality control of paper being fed into a printing press, and even the guiding or control of the operation of a printing press to the end of optimizing the printing quality and minimizing the trouble encountered in the operation of the printing press.

In the following are presented the claims, different details of the invention being allowed to vary within the inventive idea thereby defined.

**Claims**

1. A method of non-destructive measuring of the distribution of filler and/or coating materials in the thickness direction of paper, cardboard or equivalent, and the content of said filler and/or coating materials, wherein radiation emitted by an X-ray tube is used to excite the characteristic X-ray radiation of a material component to be examined, the intensity of said radiation being observed, wherein measurements are made on both sides of the paper under examination, wherein the contents of filler components and/or coating materials are determined by X-ray absorption measurements for eliminating the effects of components disturbing the observed X-ray fluorescence intensities which are used for the distribution determinations, and wherein a beta radiation absorption measurement or equivalent procedure is carried out for obtaining the basis weight of the paper under examination, characterized in that said method comprises the steps of

making a number of absorption measurements, said number of measurements being at least equal to the number of filler components and/or coating materials which have respectively different absorption coefficients, for determining the contents of the different filler components and for the coating material by constant energy X-ray radiation or radiation varying in energy in a known way during the measuring cycle;

making a number of fluorescence measurements of the characteristic X-ray radiation of the material components excited via the X-ray tube in the paper; and

determining the distribution of fillers and/or coating materials by mathematical combination of the results from said absorption and fluorescence measurements.

2. The method as claimed in claim 1, characterized in that said radiation is obtained directly from said X-ray tube.

3. The method as claimed in claim 1, characterized in that said radiation is provided by the use of a transformation target.

4. The method as claimed in any one of claims 1—3, characterized in that the contents of various filler components are measured by X-ray absorption measurements utilizing the primary radiation emitted by said X-ray tube and radiation with specific absorption properties derived from said primary radiation via a transformation target.

5. The method as claimed in any one of claims 1—4, characterized in that said method further comprises the step of measuring the attenuation by the paper under measurement of beta rays emitted by an $^{85}$Kr source to determine the basis weight in g/m$^2$ of said paper.

6. The method as claimed in any one of claims 1—5, characterized in that said measurements are carried out with at least two angles of incidence ($\alpha$) of the exciting radiation (Ie) emitted by said X-ray tube.

7. The method as claimed in any one of claims 1—6, characterized in that said measurements are carried out with at least two angles of departure (H) of the characteristic X-ray radiation excited in the paper by the radiation emitted by said X-ray tube.

8. The method as claimed in any one of claims 1—7, characterized in that the angle of incidence ($\alpha$) of said radiation is equal in magnitude to the angle of departure ($\beta$) of the excited radiation (Ie) relative to the plane of said paper (101) on the same side of said paper.

9. The method as claimed in any one of claims 1—8, characterized in that said method determines the intensity of the source radiation scattered back from the paper, which correlates with the basis weight of the paper, thereby providing an auxiliary quantity in the processing of results, in addition to X-ray fluorescence measurements.

10. The method as claimed in any one of claims 1—9, characterized in that said method examines paper in which the filler material is principally kaolin, talc, calcium carbonate and/or titanium dioxide, said method utilizing the characteristic 5.9 keV K line excited by the primary radiation source in the manganese of said transformation target, the characteristic 4.51 keV K line excited in the titanium of said transformation target and the calcium 3.69 keV K line excited in the calcium of said transformation target, and using the difference encountered between the extinction of said characteristic 5.9 keV K line in said manganese and of said characteristic 4.51 keV K line excited in said titanium primarily in determining the titanium dioxide content, utilizing the absorption difference observed in the extinctions of said K line of said titanium and said 3.69 keV K line of said calcium primarily in determining the CaCO$_3$ content, and using the information provided by the attenuation of said Ca—K line primarily for determining the combined content of talc and kaolin.

11. The method as claimed in any one of claims 1—10, characterized in that said method further comprises the step of recording the variation in intensity of the observed signal during a measuring cycle of fluorescent measurements and of absorption measurements, as a function of the variation of the energy of the radiation emitted by said X-ray tube.

12. The method as claimed in any one of claims 1—11, characterized in that said method further comprises measuring the contents of different filler components and equivalent by measuring X-ray absorption utilizing the variation during the measuring cycle of the intensity of the signal derived from the absorption measurements as a function of the variation in energy of the radiation emitted by said X-ray tube when said energy varies, so that the range of variation of the average energy covers at least the range from 3 to 8 keV.

13. The method according to any one of claims 1—12 in which the measuring of the filler distribution in the thickness-direction and the total filler content of the paper is made in on-line measurement on a paper machine.

14. The method according to any one of claims 1—12 in which feedback signals in the control system of a paper machine are produced during control of the filler distribution and/or the total content of different fillers.

15. The method according to any one of claims 1—12 in which the measuring and possibly the controlling of the coating material content and/or the distribution of the coating material, in particular its one-sidedness, is made in paper or cardboard to be coated in an on-line process or in paper processed in separate coating means.

16. The method according to any one of claims 1—12 in which quality control of the paper to be fed into a printing press is made and in which possibly guiding and/or controlling the operation of the printing press is performed.

## Patentansprüche

1. Verfahren zum zerstörungsfreien Messen der Verteilung von Füllmittel und/oder Beschichtungsmaterialien in der Dickenrichtung von Papier, Pappe oder dergleichen und des Gehalts des Füllmittels und/oder der Beschichtungsmaterialien, wobei eine von einer Röntgenröhre ausgesandte Strahlung zur Anregung der charakteristischen Röntgenstrahlung eines zu untersuchenden Materialbestandteils verwendet wird, wobei die Intensität der Strahlung beobachtet wird, wobei Messungen an beiden Seiten des Papiers bei der Untersuchung vorgenommen werden, wobei die Gehalte an Füllmaterialbestandteilen und/oder Beschichtungsmaterialien durch Röntgenabsorptionsmessungen bestimmt werden, um die Effekte von Komponenten zu eliminieren, welche die beobachteten Röntgenstrahlenfluoreszenzintensitäten stören, welche für die Verteilungsbestimmungen verwendet werden, und wobei eine Beta-Strahlungsabsorptionsmessung oder ein äquivalentes Verfahren durchgeführt wird, um das Grundgewicht des untersuchten Papiers zu ermitteln, dadurch gekennzeichnet, daß das Verfahren folgende Schritte umfaßt:

Durchführen einer Zahl von Absorptionsmessungen, wobei die Zahl der Messungen wenigstens gleich der Zahl der Füllmittelbestandteile und/oder Beschichtungsmaterialien ist, welche jeweils unterschiedliche Absorptionskoeffizienten besitzen, um die Gehalte an unterschiedlichen Füllmittelbestandteilen und/oder der Beschichtungsmaterialien durch Röntgenstrahlung mit konstanter Energie oder variierender Energie in bekannter Weise während des Meßzyklus zu bestimmen;

Durchführung einer Zahl von Fluoreszenzmessungen der charakteristischen Röntgenstrahlung der Materialbestandteile, welche in dem Papier durch die Röntgenröhre angeregt wird; und

Bestimmung der Verteilung von Füllmitteln und/oder Beschichtungsmaterialien durch mathematische Kombination der Ergebnisse aus den Absorptions- und Fluoreszenzmessungen.

2. Verfahren nach Anspruch 1, dadurch gekennzeichnet, daß die Strahlung unmittelbar aus der Röntgenröhre bezogen wird.

3. Verfahren nach Anspruch 1, dadurch gekennzeichnet, daß die Strahlung durch die Verwendung eines Transformationstargets vorgesehen wird.

4. Verfahren nach einem der Ansprüche 1 bis 3, dadurch gekennzeichnet, daß die Gehalte an verschiedenen Füllmittelbestandteilen durch Röntgenabsorptionsmessungen gemessen werden, wobei die von der Röntgenröhre ausgesandte primäre Strahlung und die Strahlung mit spezifischen Absorptionseigenschaften ausgenutzt wird, die von der primären Strahlung über ein Transformationstarget abgeleitet wird.

5. Verfahren nach einem der Ansprüche 1 bis 4, dadurch gekennzeichnet, daß zu dem Verfahren weiterhin das Messen der Schwächung von Beta-Strahlen, die von einer $^{85}$Kr-Quelle ausgesandt werden, durch das zu messende Papier gehört, um das Grundgewicht in g/m$^2$ des Papiers zu bestimmen.

6. Verfahren nach einem der Ansprüche 1 bis 5, dadurch gekennzeichnet, daß die Messungen mit wenigstens zwei Einfallswinkeln ($\alpha$) der anregenden Strahlung (le) durchgeführt werden, welche von der Röntgenröhre ausgesandt werden.

7. Verfahren nach einem der Ansprüche 1 bis 6, dadurch gekennzeichnet, daß die Messungen mit wenigstens zwei Ausfallswinkeln ($\beta$) der charakteristischen Röntgenstrahlung ausgeführt werden, welche in dem Papier durch die von der Röntgenröhre ausgesandte Strahlung angeregt wird.

8. Verfahren nach einem der Ansprüche 1 bis 7, dadurch gekennzeichnet, daß der Einfallswinkel ($\alpha$) der Strahlung in seiner Größe dem Ausfallswinkel ($\beta$) der angeregten Strahlung (le) relativ zu der Ebene des Papiers (10) auf derselben Seite des Papiers gleich ist.

9. Verfahren nach einem der Ansprüche 1 bis 8, dadurch gekennzeichnet, daß das Verfahren die Intensität der von dem Papier zurückgestreuten Quellenstrahlung bestimmt, welche mit dem Grundgewicht des Papiers korreliert, um dadurch eine Hilfsgröße bei der Verarbeitung von Ergebnissen zusätzlich zu Röntgenfluoreszenzmessungen vorzusehen.

10. Verfahren nach einem der Ansprüche 1 bis 9, dadurch gekennzeichnet, daß das Verfahren Papier untersucht, in dem das Füllmittelmaterial prinzipiell Kaolin, Talk, Kalziumcarbonat und/oder Titandioxid ist, wobei das Verfahren die 5,9 keV K-Kennlinie, die von der primären Strahlungsquelle in dem Mangan des Transformationstargets angeregt wird, die 4,51 keV K-Kennlinie, die in dem Titan des Transformationstargets angeregt wird, und die Kalzium 3,69 keV K-Kennlinie verwendet, die in dem Kalzium des Transformationstargets angeregt wird, und wobei primär zur Bestimmung des Titandioxidgehalts die ermittelte Differenz zwischen der Extinktion der 5,9 keV K-Kennlinie in dem Mangan und der 4,51 keV K-Kennlinie verwendet wird, die in dem Titan angeregt wird, wobei die beobachtete Absorptionsdifferenz bei den Extinktionen der K-Linie des Titan und der 3,69 keV K-Linie des Kalziums primär bei der Bestimmung des CaCO$_3$-Gehalts verwendet wird, und wobei die von der Schwächung der Ca—K-Linie gelieferte Information primär für die Bestimmung des kombinierten Gehaltes von Talk und Kaolin verwendet wird.

11. Verfahren nach einem der Ansprüche 1 bis 10, dadurch gekennzeichnet, daß zu dem Verfahren weiterhin der Verfahrensschritt gehört, daß die Intensitätsvariation der beobachteten Signale während eines Meßzyklusses von Fluoreszenzmessungen und von Absorptionsmessungen in Abhängigkeit von der Variation der Energie der Strahlung, die von der Röntgenröhre ausgesandt wird, aufgezeichnet wird.

12. Verfahren nach einem der Ansprüche 1 bis 11, dadurch gekennzeichnet, daß zu dem Verfahren weiterhin das Messen der Gehalte an unterschiedlichen Füllmittelbestandteilen und dergleichen durch Messung der Röntgenstrahlungsabsorption gehört, wobei während des Meßzyklus die Intensitätsvariation des von den Absorptionsmessungen abgeleiteten Signals in Abhängigkeit von der Variation der Energie der von der Röntgenröhre ausgesandten Strahlung, wenn diese Energie sich ändert, derart verwendet wird, daß der Variationsbereich der durchschnittlichen Strahlung wenigstens den Bereich von 3 bis 8 keV abdeckt.

13. Verfahren nach einem der Ansprüche 1 bis 12, dadurch gekennzeichnet, daß bei dem die Messung

## EP 0 127 650 B1

der Füllstoffverteilung in Dickenrichtung und die des gesamten Füllstoffgehaltes des Papiers mittels einer "on-line"-Messung auf einer Papiermaschine durchgeführt wird.

14. Verfahren nach einem der Ansprüche 1 bis 12, dadurch gekennzeichnet, daß die Rückführsignale bei dem Reglungssystem einer Papiermaschine während der Reglung der Füllmittelverteilung und/oder des gesamten Gehalts der unterschiedlichen Füllmittel erzeugt werden.

15. Verfahren nach einem der Ansprüche 1 bis 12, dadurch gekennzeichnet, daß die Messung und gegebenenfalls die Kontrolle des Beschichtungsmaterialgehalts und/oder die Verteilung des Beschichtungsmaterials, insbesondere dessen einseitige Anordnung, bei zu beschichtendem Papier oder bei zu beschichtender Pappe während eines "on-line"-Verfahrens oder bei Papier, das in separaten Beschichtungseinrichtungen bearbeitet wird, durchgeführt wird.

16. Verfahren nach einem der Ansprüche 1 bis 12, dadurch gekennzeichnet, daß die Qualitätskontrolle des einer Druckmaschine zuzuführenden Papiers vorgenommen wird, und daß gegebenenfalls ein Führen und/oder Regeln des Betriebs der Druckmaschine durchgeführt wird.

## Revendications

1. Procédé de mesure non destructive de la distribution de substances de remplissage et/ou de substances d'enduction dans le sens de l'épaisseur d'un papier, carton ou analogue, et de la teneur en de telles substances de remplissage et/ou de telles substances d'enduction, procédé selon lequel on utilise le rayonnement émis par un tube à rayons X pour déclencher le rayonnement X caractéristique d'un composant de la substance devant être examinée et on observe l'intensité dudit rayonnement, et selon lequel on effectue des mesures sur les deux faces du papier examiné, on détermine les teneurs en composants de la substance de remplissage et/ou en substances d'enduction par des mesures d'absorption des rayons X afin d'éliminer les effets des composants perturbant les intensités de fluorescence par les rayons X, observées, que l'on utilise pour les déterminations de distribution, et selon lequel on met en oeuvre une mesure d'absorption du rayonnement bêta ou une procédure équivalente, en vue d'obtenir le poids de base du papier examiné, caractérisé en ce que ledit procédé inclut les étapes consistant à:

réaliser un certain nombre de mesures d'absorption, ce nombre de mesures étant au moins égal au nombre des différents composants de la substance de remplissage et/ou des différentes substances d'enduction, qui possèdent des coefficients d'absorption respectivement différents, en vue de la détermination des teneurs en ces différents composants de la substance de remplissage et/ou en la substance d'enduction au moyen d'un rayonnement X d'énergie constante ou d'un rayonnement, dont l'énergie varie de façon connue pendant le cycle de mesure;

réaliser un certain nombre de mesures de fluorescence du rayonnement X caractéristique des composants des substances et excité dans le papier par l'intermédiaire du tube à rayons X; et

déterminer la distribution des substances de remplissage et/ou des substances d'enduction au moyen d'une combinaison mathématique des résultats fournis par lesdites mesures d'absorption et de fluorescence.

2. Procédé selon la revendication 1, caractérisé en ce que ledit rayonnement est fourni directement par ledit tube à rayons X.

3. Procédé selon la revendication 1, caractérisé en ce que ledit rayonnement est obtenu grâce à l'utilisation d'une cible de transformation.

4. Procédé selon l'une quelconque des revendications 1—3, caractérisé en ce que les teneurs en différents composants de remplissage sont mesurées à l'aide de mesures d'absorption des rayons X, moyennant l'utilisation du rayonnement primaire émis par ledit tube à rayons X et du rayonnement possédant des propriétés spécifiques d'absorption et dérivé dudit rayonnement primaire par l'intermédiaire d'une cible de transformation.

5. Procédé selon l'une quelconque des revendications 1—4, caractérisé en ce que ledit procédé inclut en outre l'étape de mesure de l'atténuation par le papier, au moyen d'une mesure des rayonnement téta émis par une source $^{85}$Kr, pour déterminer le poids de base en g/m$^2$ dudit papier.

6. Procédé selon l'une quelconque des revendications 1—5, caractérisé en ce que lesdites mesures sont exécutées sous au moins deux angles d'incidence (α) du rayonnement d'excitation (Ie) émis par ledit tube à rayons X.

7. Procédé selon l'une quelconque des revendications 1—6, caractérisé en ce que lesdites mesures sont exécutées avec au moins deux angles de départ (H) du rayonnement X caractéristique, déclenché dans le papier par le rayonnement émis par ledit tube à rayons X.

8. Procédé selon l'une quelconque des revendications 1—7, caractérisé en ce que l'angle d'incidence (α) dudit rayonnement possède une valeur égale à l'angle de départ (β) du rayonnement déclenché (Ie) par rapport au plan dudit papier (10), sur la même face de ce dernier.

9. Procédé selon l'une quelconque des revendications 1—8, caractérisé en ce que ledit procédé détermine l'intensité du rayonnement source diffusé par réflexion sur le papier, cette intensité étant en corrélation avec le poids de base du papier, ce qui fournit une grandeur auxiliaire lors de l'exploitation des résultats, en plus des mesures de fluorescence par les rayons X.

10. Procédé selon l'une quelconque des revendications 1—9, caractérisé en ce que ledit procédé permet d'examiner un papier, dans lequel le matériau de remplissage est principalement du kaolin, du talc, du carbonate de calcium et/ou du bioxyde de titane, ledit procédé utilisant la raie caractéristique (K) à 5,9 keV, excitée par la source de rayonnement primaire dans le manganèse de ladite cible de transformation, la raie caractéristique K à 4,51 keV, excitée dans le titane de ladite cible de transformation et la raie K à 3,69 keV du calcium, excitée dans le calcium de ladite cible de transformation, et utilisant la différence apparaissant entre l'excitation de ladite raie caractéristique K à 5,9 keV dans ledit manganèse et l'excitation de ladite raie caractéristique K à 4,51 keV, excitée dans ledit titane, principalement pour la détermination de la teneur en bioxyde de titane, et utilisant la différence d'absorption observée dans les extensions de ladite raie K du titane et ladite raie K à 3,69 keV dudit calcium, principalement pour la détermination de la teneur en $CaCO_3$, et utilisant l'information fournie par l'atténuation de ladite raie Ca—K, principalement pour la détermination de la teneur combinée en talc et kaolin.

11. Procédé selon l'une quelconque des revendications 1—10, caractérisé en ce que ledit procédé inclut en outre l'étape consistant à enregistrer la variation de l'intensité du signal observé pendant un cycle des mesures de fluorescence et des mesures d'absorption, en fonction de la variation de l'énergie du rayonnement émis par ledit tube à rayons X.

12. Procédé selon l'une quelconque des revendications 1—11, caractérisé en ce que ledit procédé comprend en outre la mesure des teneurs en différents composants de remplissage et équivalents, au moyen de la mesure de l'absorption du rayonnement X, en utilisant la variation obtenue pendant le cycle de mesure de l'intensité du signal obtenu à partir des mesures d'absorption en fonction de la variation de l'énergie du rayonnement émis par ledit tube à rayons X, lorsque ladite énergie varie, de sorte que la gamme de variation de l'énergie moyenne englobe au moins la gamme s'étendant de 3 à 8 keV.

13. Procédé selon l'une quelconque des revendications 1—12, selon lequel la mesure de la distribution de la substance de remplissage dans la direction de l'épaisseur et de la teneur totale en substance de remplissage du papier est exécutée selon une mesure en ligne dans une machine à papier.

14. Procédé selon l'une quelconque des revendications 1—12, selon lequel des signaux de réaction dans le système de commande d'une machine à papier sont produits pendant le réglage de la distribution de la substance de remplissaage et/ou de la teneur totale en différentes substances de remplissage.

15. Procédé selon l'une quelconque des revendications 1—12, selon lequel la mesure et éventuellement le réglage de la teneur en substance d'enduction et/ou de la distribution de la substance d'enduction, en particulier son caractère unilatéral, sont exécutés dans du papier ou du carton devant être enduit selon un procédé en continu ou bien dans du papier traité avec des moyens séparés d'enduction.

16. Procédé selon l'une quelconque des revendications 1—12, selon lequel on réalise un contrôle de la qualité du papier à introduire dans une presse d'imprimerie, et selon lequel on exécute éventuellement un guidage et/ou un réglage du fonctionnement de la presse d'imprimerie.

FIG.1

FIG.2

EP 0 127 650 B1

EP 0 127 650 B1

FIG.3

FIG.4A          FIG.4B

2

FIG. 5A

FIG. 5B

FIG. 5C

FIG.6A

FIG.6B

4

FIG.7A

FIG.7B

FIG.7C

FIG.7D

X-RAY TUBE POWER SOURCE

VOLTAGE SOURCE

AMPLIFIER
ANALYZER
COUNTER

CONTROL UNIT

COMPUTER

MULTI-CHANNEL COUNTER

DETECTOR

PRE-AMPLIFIER